# FASCICULE DE BREVET EUROPEEN

(11) **EP 2 235 202 B1**
(45) Date de publication et mention de la délivrance du brevet: **19.06.2013**
(21) Numéro de dépôt: 09704180.0
(22) Date de dépôt: 20.01.2009
(51) Int. Cl.: C12Q 1/04

(54) **PROCÉDÉ DE DÉTECTION ET/OU D'IDENTIFICATION DE CLOSTRIDIUM DIFFICILE**
MEDIUM FÜR DEN NACHWEIS UND/ODER DIE IDENTIFIKATION VON CLOSTRIDIUM DIFFICILE
METHOD FOR DETECTING AND/OR IDENTIFYING CLOSTRIDIUM DIFFICILE

(30) Priorité: 21.01.2008 FR 0850354
(43) Date de publication de la demande: 06.10.2010
(73) Titulaire: Biomérieux, 69280 Marcy L'etoile (FR)
(72) Inventeur: HALIMI, Diane, F-01700 Saint-Maurice-de-Beynost (FR); PERRY, John, Newcastle-upon-tyne NE7 7BH (GB); ORENGA, Sylvain, F-01160 Neuville sur Ain (FR); ASIR, Kerry, Dudley Northumberland NE23 7HU (GB); BOURGUIGNON, Marie-Pierre, F-38390 Vertrieux (FR)
(86) Numéro de dépôt international: PCT/FR2009/050072
(87) Numéro de publication internationale: WO 2009/092982

(56) Documents cités:
- US-A- 5 635 367
- US-A1- 2007 004 021
- US-B1- 6 287 798
- REYES ROMINA C ET AL: "Performance of TechLab C. DIFF QUIK CHEK and TechLab C. DIFFICILE TOX A/B II for the detection of Clostridium difficile in stool samples." DIAGNOSTIC MICROBIOLOGY AND INFECTIOUS DISEASE SEP 2007, vol. 59, no. 1, septembre 2007 (2007-09), pages 33-37, XP022249974 ISSN: 0732-8893
- RUSSMANN H ET AL: "Evaluation of three rapid assays for detection of Clostridium difficile toxin A and toxin B in stool specimens" EUROPEAN JOURNAL OF CLINICAL MICROBIOLOGY AND INFECTIOUS DISEASES 200702 DE, vol. 26, no. 2, février 2007 (2007-02), pages 115-119, XP002493466 ISSN: 0934-9723

## Description

La présente invention concerne l'utilisation d'un milieu réactionnel spécifique de *Clostridium difficile.* Elle concerne également un procédé de détection et/ou d'identification des *Clostridium difficile* mettant en oeuvre cette utilisation.

*Clostridium difficile* est un germe commensal de la flore digestive. On retrouve des spores du *Clostridium difficile* dans le sol, dans les hôpitaux, la forme active ne se retrouvant qu'au niveau intestinal. Au microscope, après coloration de Gram, ce sont des bacilles allongés avec une extrémité légèrement renflée. Etant résistant à la plupart des antibiotiques, il peut alors se développer de façon importante en cas de perturbation de la flore digestive par antibiothérapie. Il secrète alors deux toxines A et B, une entérotoxine et une cytotoxine, à l'origine de colites pseudo-membraneuses ou de diarrhées post-antibiotiques. De ce fait, *Clostridium difficile* est actuellement reconnu comme un entéropathogène majeur, majoritairement impliqué dans les diarrhées nosocomiales de l'adulte.
Le diagnostic repose sur différentes techniques telles que la détection de l'activité toxine (cytotoxicity activity, CTA). Toutefois, cette technique présente des inconvénients, telles que notamment sa durée de mise en oeuvre, et la nécessité de l'expertise de techniciens pour la lecture des résultats.
Le diagnostic peut également être mis en oeuvre par immunologie, grâce à un test de type ELISA. Il est toutefois recommandé d'associer les techniques immunologiques utilisées pour la détection des toxines à la culture bactérienne pour isoler les souches de *Clostridium difficile.* Reyes et al. (Diagnostic Microbiology and Infectious Disease, 2007 ; 59 : 33-37) décrivent des kits d'immuno-détection de *C. difficile* évalués en comparaison avec la culture des échantillons de selles. De la même façon, Rüssmann et al. (Eur. J. Clin. Microbiol. Infect. Dis, 2007 ; 26 : 115-119) contrôlent la détection des toxines de *C. difficile* dans des échantillons cliniques par une culture en milieu gélosé. L'étape de culture peut être mise en oeuvre sur une gélose *Clostridium difficile* commercialisée par la demanderesse, les colonies de *Clostridium difficile* étant ensuite identifiées par des méthodes biochimiques telles que la galerie api 20 A ou la galerie rapid ID 32 A. La confirmation par l'utilisation de la galerie Rapid ID 32A est mise en oeuvre par Rüssmann *et al.* (*supra*). Toutefois, l'identification par galerie nécessite d'avoir une culture pure, de la bactérie à identifier, sur un milieu de croissance adapté afin d'obtenir suffisamment de biomasse (3 à 4 Mc Farland) ce qui prend, au mieux, 48h (culture d'isolement de 24 à 72 heures à partir du prélèvement donnant uniquement la détection présomptive plus culture de 24 à 72 heures pour générer suffisamment de biomasse). La lecture de la galerie s'effectue ensuite après 4h d'incubation pour la Rapid ID 32 A et 24 à 48h pour la galerie 20A.
Enfin, le diagnostic peut être réalisé par des techniques de biologie moléculaire. Ces techniques ne sont toutefois pas utilisées en routine à ce jour.
D'une manière surprenante, les inventeurs ont mis en évidence que l'utilisation de substrat(s) enzymatique(s) de beta-glucosidase permet une identification aisée et rapide de *Clostridium difficile*. Ce résultat est complètement inattendu puisque le test beta-glucosidase de la galerie rapid ID 32 A est négatif pour cette espèce. Le milieu selon l'invention permet une détection et une identification définitive à partir du prélèvement dès 24h.

Avant de présenter l'invention, les définitions suivantes sont données pour permettre de mieux comprendre l'invention. Elles ne sont nullement limitatives.
Par milieu réactionnel, on entend un milieu comprenant tous les éléments nécessaires à l'expression d'un métabolisme et/ou à la croissance de microorganismes. Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine, de l'agarose ou d'autres gélifiants naturels ou artificiels. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press). Le milieu réactionnel selon l'invention doit permettre la croissance des *C. difficile.*
Le milieu réactionnel peut comprendre un ou plusieurs éléments en combinaison, tels que des acides aminés, des peptones, des hydrates de carbone, des nucléotides, des minéraux, des vitamines... Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant, un ou plusieurs indicateurs métaboliques, un ou plusieurs régulateurs métaboliques...
Le milieu réactionnel peut être un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture.
L'homme du métier peut également utiliser une bi-boite, permettant de comparer aisément deux milieux, comprenant différents substrats ou différents mélanges sélectifs, sur lesquels on aura déposé un même échantillon biologique.
Le milieu réactionnel peut comprendre un ou plusieurs inducteurs de β-glucosidase.
Par inducteur de beta-glucosidase, on entend un composé induisant une augmentation de l'expression de l'activité métabolique ciblée, toute condition expérimentale étant égale par ailleurs, l'activité métabolique est plus forte lorsque l'inducteur est à une concentration appropriée que lorsqu'il est absent ou à une concentration inadaptée.
On peut citer notamment un hydrate de carbone constitué d'un radical lié en position β au glucose ou un hydrate de carbone avec une sous-unité β-glucoside, en particulier le cellobiose, la cellulose, l'amidon, le cellotriose, le trehalose, le Méthyl-β-glucoside. Sans être limitatif, une concentration comprise entre 100ng/l et 10g/l, préférentiellement comprise entre 10mg/l et 3g/l est particulièrement adaptée a la présente invention..
Le milieu réactionnel peut comprendre un ou plusieurs activateurs de croissance des souches de *C. difficile.*
Par activateur de croissance, on entend un composé ou un groupe de composés qui stimule la croissance de microorganismes. On peut citer notamment le sang, sérum, jaune d'oeufs.
Sans être limitatif, une concentration comprise entre 0,1 et 10 % est particulièrement adaptée à la présente invention.
Le milieu réactionnel peut comprendre un ou plusieurs agents réducteurs.
Par agent réducteur, on entend un composé ou un groupe de composés qui facilite la croissance des germes anaérobies en neutralisant l'O₂ dissout présent dans le milieu. On peut citer notamment la Cystéine, le Pyruvate, l'Oxyrase, le Sulfite de sodium, la Dithionite, l'Histidine, le Sulfide ferreux.

Sans être limitatif, une concentration comprise entre 0,05 et 50g/l, préférentiellement entre 0,1 et 2g/l est particulièrement adaptée à la présente invention.
Le milieu réactionnel peut comprendre un ou plusieurs inducteurs de la germination des spores de *C. difficile.*
Par inducteur de la germination des spores, on entend un composé ou un groupe de composé qui favorise le passage de l'état de spore à l'état végétatif des *C. difficile.* On peut citer notamment le Taurocholate de Sodium.
Sans être limitatif, une concentration comprise entre 0,1 et 10 g/l, préférentiellement entre 1 et 5 g/l est particulièrement adaptée à la présente invention.
Le milieu réactionnel peut comprendre un ou plusieurs agents sélectifs.
Par agent sélectif, on entend tout composé susceptible d'empêcher ou de ralentir la croissance d'un microorganisme. Sans être limitatif, une concentration comprise entre 5 mg/l et 5 g/l est particulièrement adaptée à la présente invention.
On peut citer comme agent sélectif, un antibiotique tel que la D-cyclosérine, les céphalosporines, telles que la Céfoxitine ou le Céfotaxime, la Colistine, la Polymixine, la Fosfomycine, la Tobramycine, la Gentamicine, l'Aztréonam, le Triméthoprime, les quinolones telles que l'acide Nalidixique, un antifongique tel que notamment l'amphotéricine B, le fluconazole ou l'itraconazole.
Par antibiotique, on entend tout composé susceptible d'empêcher ou de ralentir la croissance d'une bactérie. Ils appartiennent notamment aux groupes des céphalosporines, des aminosides, des polypeptides, des sulfamides, des quinolones. A titre indicatif, on peut citer notamment les antibiotiques cefotaxime, ceftazidime, cefoxitine, ceftriaxone, cefpodoxime, aztréonam, Triméthoprime, tobramycine, moxalactam, fosfomycine, D-cylcosérine, Polymixine, Colistine.
Par antifongique, on entend tout composé susceptible d'empêcher ou de ralentir la croissance d'une levure ou d'une moisissure. A titre indicatif, on peut citer notamment l'amphotéricine B, le fluconàzole, l'itraconazole, le voriconazole, la cycloheximide.
Le milieu réactionnel peut comprendre un deuxième substrat enzymatique, tel que notamment un substrat d'osidase, d'estérase, de peptidase, .... IL peut s'agir d'un substrat ciblant une enzyme exprimée par *Clostridium difficile* telle que la Proline aminopeptidase ou d'une enzyme non exprimée par *C. difficile* telle que la β-ribosidase.

Par substrat, on entend une molécule pouvant être hydrolysée par une enzyme, telle que la beta glucosidase en un produit permettant la détection, directe ou indirecte d'un microorganisme. Ce substrat comprend notamment une première partie spécifique de l'activité enzymatique à révéler et une seconde partie faisant office de marqueur, ci-après appelée partie marqueur. Cette partie marqueur peut être chromogène, fluorogène, luminescente...
Par substrat de beta glucosidase, apte à l'identification de *C. difficile,* on entend un substrat qui induit, dans des conditions de croissance adaptées, la coloration de *C. difficile.* De tels substrats peuvent notamment être sélectionnés grâce au test décrit dans l'exemple 1.
On entend notamment le 2-Hydroxyphényl-β-glucoside (Catéchol-β-glucoside); Magenta-β-glucoside (5-Bromo-6-chloro-3-indoxyl-β-glucoside); DHF-β-glucoside (Dihydroxyflavone-β-glucoside); l'Esculine (Esculétine-β-glucoside); CHE-β-glucoside (3,4-Cyclohexénoesculétine-β-glucoside); 8-Hydroxiquinoline-β-glucoside; X-β-glucoside (5-Bromo-4-chloro-3-indoxyl-β-glucoside) ; Rose-β-glucoside (6-Chloro-3-indoxyl-β-glucoside) ; 6-Bromo-3-indoxyl-β-glucoside ; Blue-β-glucoside (5-Bromo-3-indoxyl-β-glucoside) ; 6-Fluoro-3-indoxyl-β-glucoside ; Alizarine-β-glucoside ; (P)-Nitrophényl-β-glucoside ; 4-Méthylumbelliferyl-β-glucoside, Naphtholbenzein-β-glucoside, Indoxyl-N-méthyl-β-glucoside, 5-Bromo-4-chloro-3-indoxyl-N-méthyl-β-glucoside, le Naphtyl-β-glucoside ; l'Aminophényl-β-glucoside ; le Dichloroaminophényl-β-glucoside.
Préférentiellement, le substrat de beta glucosidase, apte à l'identification de *C. difficile,* est choisi parmi 2-Hydroxyphényl-β-glucoside (Catéchol-β-glucoside); Magenta-β-glucoside (5-Bromo-6-chloro-3-indoxyl-β-glucoside); Dihydroxyflavone-β-glucoside ; l'Esculine (Esculétine-β-glucoside), 3,4-Cyclohexenoesculetine-B-glucoside et l'Alizarine-β-glucoside.
Préférentiellement, la concentration en substrat de beta glucosidase dans le milieu selon l'invention est comprise entre 25 et 1000mg/l et plus préférentiellement entre 50 et 400mg/l.
Le milieu réactionnel peut comprendre un deuxième substrat enzymatique ou plusieurs substrats enzymatiques, préférentiellement choisi parmi un substrat d'osidase, d'estérase ou de peptidase.

Par sustrat d'osidase, on entend notamment les substrats de d'alpha-glucosidase, de galactosidase, de ribosidase, d'hexosaminidase, de glucuronidase, de xylosidase, de fucosidase, de cellobiosidase, d'arabinosidase, de mannosidase.
Par riboside on entend ribofuranoside et/ou ribopyranoside, par arabinoside on entend arabinofuranoside et/ou arabinopyranoside, par fucoside on entend D-fucoside et/ou L-fucoside. Quand ce n'est pas précisé, il peut s'agir soit de l'α-oside, soit du β-oside. On entend notamment le 5-Bromo-6-chloro-3-indoxyl- α-glucoside ; Dihydroxyflavone-α-glucoside ; 3,4-Cyclohexénoesculétine- α-glucoside ; 8-Hydroxiquinoline-α-glucoside ; 5-Bromo-4-chloro-3-indoxyl- α-glucoside ; 6-Chloro-3-indoxyl-α-glucoside ; 5-Bromo-3-indoxyl- α-glucoside ; Alizarine- α-glucoside ; Nitrophényl-α-glucoside ; 4-Méthylumbelliferyl- α-glucoside ; Naphtholbenzein- α-glucoside ; Indoxyl-N-méthyl- α-glucoside ; 5-Bromo-4-chloro-3-indoxyl-N-méthyl- α-glucoside ; Naphtyl- α-glucoside ; Aminophényl- α-glucoside ; Dichloroaminophényl- α-glucoside ; 2-Hydroxyphényl-galactoside ; 5-Bromo-6-chloro-3-indoxyl- galactoside ; Dihydroxyflavone- galactoside ; 3,4-Cyclohexénoesculétine- galactoside ; 8-Hydroxiquinoline- galactoside ; 5-Bromo-4-chloro-3-indoxyl- galactoside ; 4-Chloro-3-indoxyl- galactoside ; 6-Chloro-3-indoxyl- galactoside ; 6-Bromo-3-indoxyl-galactoside ; 5-Bromo-3-indoxyl- galactoside ; 6-Fluoro-3-indoxyl- galactoside ; Alizarine- galactoside ; Nitrophényl- galactoside ; 4-Mèthylumbelliferyl- galactoside ; Naphtholbenzein- galactoside ; Indoxyl-N-méthyl- galactoside ; 5-Bromo-4-chloro-3-indoxyl-N-méthy- galactoside ; Naphtyl- galactoside ; Aminophényl- galactoside ; Dichloroaminophényl- galactoside ; 2-Hydroxyphényl-riboside ; 5-Bromo-6-chloro-3-indoxyl- riboside ; Dihydroxyflavone- riboside ; 5-Bromo-4-chloro-3-indoxyl-riboside ; 6-Chloro-3-indoxyl- riboside ; 5-Bromo-3-indoxyl- riboside ; 6-Fluoro-3-indoxyl- riboside ; Alizarine- riboside ; Nitrophényl- riboside ; 4-Méthylumbelliferyl-riboside ; Naphtyl- riboside ; Aminophényl- riboside ; Dichloroaminophényl- riboside ; 2-Hydroxyphényl-N-acétyl-glucosaminide ; 5-Bromo-6-chloro-3-indoxyl-N-acétyl-glucosaminide ; Dihydroxyflavone- N-acétyl-glucosaminide ; 3,4-Cyclohexénoesculétine- N-acétyl-glucosaminide; 5-Bromo-4-chloro-3-indoxyl-N-acétyl-glucosaminide ; 6-Chloro-3-indoxyl- N-acétyl-glucosaminide ; 6-Bromo-3-indoxyl- N-acétyl-glucosaminide ; 5-Bromo-3-indoxyl- N-acétyl-glucosaminide ; 6-Fluoro-3-indoxyl- N-acétyl-glucosaminide ; Alizarine-N-acétyl-glucosaminide ; Nitrophényl-N-acétyl-glucosaminide ; 4-Méthylumbelliferyl- N-acétyl-glucosaminide ; 5-Bromo-4-chloro-3-indoxyl-N-méthyl- N-acétyl-glucosaminide ; Naphtyl- N-acétyl-glucosaminide ; Aminophényl- N-acétyl-glucosaminide ; Dichloroaminophényl-N-acétyl-glucosaminide ; 5-Bromo-6-chloro-3-indoxyl- glucuronide ; Dihydroxyflavone-glucuronide ; 3,4-Cyclohexénoesculétine- glucuronide ; 8-Hydroxiquinoline-glucuronide ; 5-Bromo-4-chloro-3-indoxyl- glucuronide ; 6-Chloro-3-indoxyl-glucuronide ; 6-Bromo-3-indoxyl- glucuronide ; 5-Bromo-3-indoxyl- glucuronide ; 6-Fluoro-3-indoxyl- glucuronide ; Alizarine- glucuronide ; Nitrophényl- glucuronide ; 4-Méthylumbelliferyl- glucuronide ; Naphtholbenzein- glucuronide ; Indoxyl-N-méthyl-glucuronide ; 5-Bromo-4-chloro-3-indoxyl-N-méthyl- glucuronide ; Naphtyl-glucuronide ; Aminophényl- glucuronide ; Dichloroaminophényl- glucuronide ; 5-Bromo-6-chloro-3-indoxyl- xyloside ; Dihydroxyflavone- xyloside ; 5-Bromo-4-chloro-3-indoxyl- xyloside ; 6-Chloro-3-indoxyl- xyloside ; 5-Bromo-3-indoxyl-xyloside ; Alizarine- xyloside ; Nitrophényl- xyloside ; 4-Méthylumbelliferyl- xyloside ; Naphtyl- xyloside ; Aminophényl- xyloside ; Dichloroaminophényl- xyloside ; 5-Bromo-6-chloro-3-indoxyl- fucoside ; Dihydroxyflavone- fucoside ; 3,4-Cyclohexénoesculétine-fucoside ; 5-Bromo-4-chloro-3-indoxyl- fucoside ; 6-Chloro-3-indoxyl- fucoside ; 5-Bromo-3-indoxyl- fucoside ; Alizarine- fucoside ; Nitrophényl- fucoside ; 4-Méthylumbelliferyl- fucoside ; Naphtyl- fucoside ; Aminophényl- fucoside ; Dichloroaminophényl- fucoside ; 2-Hydroxyphényl-cellobioside ; 5-Bromo-6-chloro-3-indoxyl- cellobioside ; Dihydroxyflavone- cellobioside ; 3,4-Cyclohexénoesculétine-cellobioside ; 8-Hydroxiquinoline- cellobioside ; 5-Bromo-4-chloro-3-indoxyl-cellobioside ; 6-Chloro-3-indoxyl- cellobioside ; 6-Bromo-3-indoxyl- cellobioside ; 5-Bromo-3-indoxyl- cellobioside ; 6-Fluoro-3-indoxyl- cellobioside ; Alizarine-cellobioside ; Nitrophényl- cellobioside ; 4-Méthylumbelliferyl- cellobioside ; Naphtholbenzein- cellobioside ; Indoxyl-N-méthyl- cellobioside ; 5-Bromo-4-chloro-3-indoxyl-N-méthyl- cellobioside ; Naphtyl- cellobioside ; Aminophényl- cellobioside ; Dichloroaminophényl- cellobioside ; 5-Bromo-6-chloro-3-indoxyl- arabinoside ; Dihydroxyflavone- arabinoside ; 5-Bromo-4-chloro-3-indoxyl- arabinoside ; 6-Chloro-3-indoxyl- arabinoside ; 5-Bromo-3-indoxyl- arabinoside ; Alizarine- arabinoside ; Nitrophényl- arabinoside ; 4-Méthylumbelliferyl- arabinoside ; 5-Bromo-4-chloro-3-indoxyl-N-méthyl- arabinoside ; Naphtyl- arabinoside ; Aminophényl- arabinoside ; Dichloroaminophényl- arabinoside ; 5-Bromo-6-chloro-3-indoxyl- mannoside ; Dihydroxyflavone- mannoside ; 3,4-Cyclohexénoesculétine- mannoside ; 5-Bromo-4-chloro-3-indoxyl- mannoside ; 4-Chloro-3-indoxyl- mannoside ; 6-Chloro-3-indoxyl-mannoside ; 6-Bromo-3-indoxyl- mannoside ; 5-Bromo-3-indoxyl- mannoside ; Alizarine- mannoside ; Nitrophényl- mannoside ; 4-Méthylumbelliferyl- mannoside ; Indoxyl-N-méthyl- mannoside ; 5-Bromo-4-chloro-3-indoxyl-N-méthyl- mannoside ; Naphtyl- mannoside ; Aminophényl- mannoside; Dichloroaminophényl- mannoside. Sans être limitatif, une concentration comprise entre 25 et 1000mg/l est particulièrement adaptée à la présente invention.
Préférentiellement, le substrat d'osidase est choisi parmi les substrats d'alpha-galactosidase, de beta galactosidase, d'hexosaminidase et de cellobiosidase et notamment parmi : 5-Bromo-4-chloro-3-indoxyl- galactoside ; 5-Bromo-6-chloro-3-indoxyl-N-acétyl-glucosaminide ; 3,4-Cyclohexénoesculétine- cellobioside ; 5-Bromo-4-chloro-3-indoxyl- cellobioside.
Par substrat d'estérase, on entend notamment 5-Bromo-6-chloro-3-indoxyl- octanoate ; Dihydroxyflavone- octanoate ; 5-Bromo-4-chloro-3-indoxyl- octanoate ; 6-Chloro-3-indoxyl- octanoate ; 6-Bromo-3-indoxyl- octanoate ; 5-Bromo-3-indoxyl- octanoate ; 6-Fluoro-3-indoxyl- octanoate ; Alizarine- octanoate ; Nitrophényl- octanoate ; 4-Méthylumbelliferyl- octanoate ; Naphtyl- octanoate ; Aminophényl- octanoate ; Dichloroaminophényl- octanoate ; 5-Bromo-6-chloro-3-indoxyl- butyrate ; 5-Bromo-4-chloro-3-indoxyl- butyrate ; 6-Chloro-3-indoxyl- butyrate ; 6-Bromo-3-indoxyl-butyrate ; 5-Bromo-3-indoxyl-butyrate ; Alizarine- butyrate ; Nitrophényl- butyrate ; 4-Méthylumbelliferyl- butyrate ; Naphtyl- butyrate ; Aminophényl- butyrate ; Dichloroaminophényl- butyrate ; 5-Bromo-6-chloro-3-indoxyl- nonanoate ; 5-Bromo-4-chloro-3-indoxyl- nonanoate ; 6-Chloro-3-indoxyl- nonanoate ; 6-Bromo-3-indoxyl-nonanoate ; 5-Bromo-3-indoxyl- nonanoate ; Alizarine- nonanoate ; Nitrophényl-nonanoate ; 4-Méthylumbelliferyl- nonanoate ; Naphtyl- nonanoate ; Aminophényl-nonanoate ; Dichloroaminophényl- nonanoate ; 5-Bromo-6-chloro-3-indoxyl-phosphate ; Dihydroxyflavone- phosphate ; 3,4-Cyclohexénoesculétine- phosphate ; 5-Bromo-4-chloro-3-indoxyl- phosphate ; 6-Chloro-3-indoxyl- phosphate ; 6-Bromo-3-indoxyl- phosphate ; 5-Bromo-3-indoxyl- phosphate ; 6-Fluoro-3-indoxyl- phosphate ; Alizarine- phosphate ; Nitrophényl- phosphate ; 4-Méthylumbelliferyl- phosphate ; Naphtholbenzein- phosphate ; Indoxyl-N-méthyl- phosphate ; 5-Bromo-4-chloro-3-indoxyl-N-méthyl- phosphate ; Naphtyl- phosphate ; Aminophényl- phosphate ; Dichloroaminophényl- phosphate. Sans être limitatif, une concentration comprise entre. 25 et 1000 mg/l est particulièrement adaptée à la présente invention.
Préférentiellement, le substrat d'estérase est choisi parmi les substrats de butyrate estérase, de lipase et de phosphatase et notamment parmi : 5-Bromo-6-chloro-3-indoxyl-octanoate ; 5-Bromo-4-chloro-3-indoxyl- butyrate ; 5-Bromo-3-indoxyl- nonanoate et 5-Bromo-6-chloro-3-indoxyl- phosphate.
Par substrat de peptidase, on entend notamment les substrats de Proline-aminopeptidase, d'Alanine-aminopeptdidase, de Leucine-aminopeptidase, de Pyrrolidonyl-arylamidase, de Phenylalanine-aminopeptidase, d'Ala-Phe-Pro-peptidase de Tyrosine-aminopeptidase. Ces substrats sont notamment les composés associant via une liaison peptidique l'acide aminé ou le peptide correspondant et un radical p-Nitroaniline, 7-Aminométhylcoumarine, 7-Aminophénoxazinone, Naphtylamine, Aminophényl, Dichloroaminophényl. On peut citer notamment le Prolyl-p-nitroanilide, le Prolyl-7-amido-4-méthyl-coumarine, le Prolyl-dichloroamidophénol, l'Alanyl-dichloroamidophénol, le Leucyl-p-nitroanilide, le Pyrrolidonyl-7-amido-4-méthylcoumarine, le Phénylalanyl-p-nitroanilide, l'Ala-Phe-Pro-7-amido-1-pentyl-phénoxazine-3-one, le Tyrosyl-dichloro-amidophénol. Sans être limitatif, une concentration comprise entre 25 et 1000mg/l est particulièrement adaptée à la présente invention.
Préférentiellement, le substrat de peptidase est choisi parmi les substrats de Proline-aminopeptidase (ou Prolyl-arylamidase), de Leucine-aminopeptidase (ou Leucyl-arylamidase) et de Pyrrolidonyl-arylamidase et notamment parmi : Prolyl-p-nitroanilide, le Prolyl-7-amido-4-méthyl-coumarine, Leucyl-p-nitroanilide et Pyrrolidonyl-7-amido-4-méthylcoumarine.
Par échantillon biologique,on entend un échantillon clinique, issu d'un prélèvement de liquide biologique ou un échantillon alimentaire, issu de tout type d'aliment. Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits, de yaourt, de viande, d'oeufs, de légumes, de mayonnaise, de fromage ; de poisson..., un échantillon alimentaire issu d'une alimentation destinée aux animaux, tel que notamment un échantillon issu de farines animales.

A ce titre, l'invention concerne un procédé de détection et/ou d'identification de *Clostridium difficile,* caractérisé en ce qu'il comprend les étapes suivantes :
a) disposer d'un milieu réactionnel comprenant au moins un substrat de beta glucosidase, apte à l'identification de *C. difficile,*
b) ensemencer le milieu avec un échantillon biologique à tester,
c) laisser incuber, et
d) détecter l'hydrolyse du substrat de beta-glucosidase indiquant la présence de *Clostridiurn difficile*

Selon un mode préféré de réalisation de l'invention, lors de l'étape c), l'incubation est effectuée en anaérobiose.
Selon un mode préféré de réalisation de l'invention, ledit substrat de beta-glucosidase est choisi parmi l'Alizarine-β-glucoside, le Magenta-β-glucoside (5-Bromo-6-chloro-3-indoxyl-β-glucoside) et le CHE-β-Glucoside (3,4-Cyclohexenocsculetinc-β-glucoside). Préférentiellement, ledit substrat de beta-glucosidase est à une concentration comprise entre 25 et 1000mg/l, plus préférentiellement entre 50 et 400mg/l
Selon un mode préféré de réalisation de l'invention, ledit milieu réactionnel comprend en outre, de l'esculine. Préférentiellement, l'esculine est à une concentration comprise entre 5 et 500 mg/l, préférentiellement entre 10 et 100 mg/l.
Selon un mode préféré de réalisation de l'invention, ledit milieu réactionnel comprend en outre, au moins un inducteur de β-glucosidase, préférentiellement choisi parmi le cellobiose ou le Methyl-β-glucoside.
Selon un mode préféré de réalisation de l'invention, ledit milieu réactionnel comprend en outre, au moins un activateur de croissance des souches de *C. difficile,* préférentiellement choisi parmi le sang, sérum, un agent réducteur tel que notamment la cystéine, le Pyruvate, le Sulfide ferreux.
Selon un mode préféré de réalisation de l'invention, ledit milieu réactionnel comprend en outre, au moins un inducteur de la germination des spores de *C. difficile,* préférentiellement le Taurocholate de Sodium.
Selon un mode préféré de réalisation de l'invention, ledit milieu réactionnel comprend en outre au moins un agent sélectif, préférentiellement choisi parmi, un antibiotique, tel que notamment la D-cyclosérine, la céfoxitine, le céfotaxime, un antifongique, tel que notamment l'amphotéricine B, le fluconazole, l'itraconazole ou le voriconazole.
Selon un mode préféré de réalisation de l'invention, ledit milieu réactionnel comprend en outre au moins un deuxième substrat enzymatique, préférentiellement choisi parmi un substrat d'osidase, d'estérase, de peptidase.
Selon un mode préféré de réalisation de l'invention, ledit milieu réactionnel comprend en outre au moins un agent réducteur, préférentiellement la cystéine.

L'invention concerne également l'utilisation d'un milieu réactionnel comprenant au moins un substrat de beta-glucosidase, apte à l'identification de *C. difficile,* pour la détection et/ou l'identification de *Clostridium difficile.*
Selon un mode préféré de réalisation de l'invention, ledit substrat de beta-glucosidase est choisi parmi l'Alizarine-β-glucoside, le Magenta-β-glucoside (5-Bromo-6-chloro-3-indoxyl-β-glucoside) et le CHE-β-Glucoside (3,4-Cyclohexenoesculetine-β-glucoside) Préférentiellement, ledit substrat de beta-glucosidase est à une concentration comprise entre 25 et 1000mg/l et plus préférentiellement entre 50 et 400mg/l.
Selon un mode préféré de réalisation de l'invention, ledit milieu réactionnel comprend, en outre, de l'esculine.
Préférentiellement, l'esculine est à une concentration comprise entre 5 et 500mg/l, préférentiellement entre 10 et 100 mg/l.
Selon un mode préféré de réalisation de l'invention, ledit milieu réactionnel comprend, en outre, au moins un inducteur de β-glucosidase, préférentiellement choisi parmi le cellobiose ou le Methyl-β-glucoside.
Selon un mode préféré de réalisation de l'invention, ledit milieu réactionnel comprend, en outre, au moins un activateur de croissance des souches de *C. difficile,* préférentiellement choisi parmi le sang, du sérum, un agent réducteur tel que notamment la Cystéine, le Pyruvate, le Sulfide ferreux.
Selon un mode préféré de réalisation de l'invention, ledit milieu réactionnel comprend, en outre, au moins un inducteur de la germination des spores de *C. difficile,* préférentiellement le Taurocholate de Sodium.
Selon un mode préféré de réalisation de l'invention, ledit milieu réactionnel comprend, en outre, au moins un agent sélectif. Préférentiellement, l'agent sélectif est choisi parmi, un antibiotique, préférentiellement la D-cyclosérine, la céfoxitine ou le céfotaxime, un antifongique préférentiellement l'amphotéricine B, le fluconazole, l'itraconazole ou le voriconazole.
Selon un mode préféré de réalisation de l'invention, ledit milieu réactionnel comprend, en outre, au moins un deuxième substrat enzymatique. Préférentiellement, ledit deuxième substrat est un substrat d'osidase, d'estérase ou de peptidase.
Selon un mode préféré de réalisation de l'invention, ledit milieu réactionnel comprend en outre au moins un agent réducteur, préférentiellement la cystéine.

Les exemples ci-dessous sont donnés à titre explicatif et n'ont aucun caractère limitatif. Ils permettront de mieux comprendre l'invention.

### Exemple 1 - Identifcation de substrats de beta glucosidase aptes à l'identification de C. difficile

Sur la base de la présente invention, les substrats de β-glucosidase adaptés à la détection des souches de *Clostridium difficile* peuvent être identifiés par l'homme du métier grâce à l'exemple ci dessous.
Le substrat de β-glucosidase potentiel est testé en l'incorporant à un milieu permettant la croissance de *Clostridium difficile,* tel qu'un milieu sélectif, contenant par exemple des inhibiteurs pour ralentir la croissance de certaines bactéries ou de certains eucaryotes ou un milieu non sélectif, tels que la Gélose Columbia en flacon (réf 41244) ou une base à l'oeuf selon George *et al.* (voir exemple 2).
En fonction du substrat de β-glucosidase à tester, il peut être nécessaire d'ajouter un réactif additionnel, tel que notamment un sel de Fer, un dérivé d'α-Naphtol, pour révéler le produit d'hydrolyse.

Préférentiellement, le substrat à tester est testé à une concentration comprise entre 25 et 1000 mg/l. Le milieu ainsi réalisé est réparti dans un consommable, par exemple une boîte de Petri ou un tube. Une collection de souches comprenant au moins une souche de *C. difficile* est ensemencée, préférentiellement une seule souche par milieu. Le milieu est alors incubé en anaérobiose à une température appropriée, généralement comprise entre 20 et 50°C, préférentiellement entre 30 et 40°C.
Les substrats adaptés sont ceux pour lesquels une hydrolyse par la ou les souches de *C. difficile* est détectée après incubation. La détection est réalisée en observant une variation des propriétés optiques (colonies et/ou milieu coloré ou fluorescent) soit visuellement sous lumière naturelle, artificielle ou sous UV, soit à l'aide d'un appareil adapté, notamment spectrophotomètre, fluorimètre, luminomètre, analyseur d'images.

### Exemple 2

Des souches du genre *Clostridium spp* ont été testées sur 6 milieux différents contenant chacun un substrat chromogénique de beta-glucosidase. La lecture des boites est ensuite effectuée à 48h afin de déterminer la spécificité de chaque substrat.

### 1. MILIEU ET MICROORGANISMES

La base des milieux est une base Egg Yolk selon George et al. dont la composition est la suivante (en g/l) :

| | |
|---|---|
| Proteose peptone no. 3 | 40 |
| Di sodium hydrogen phosphate | 5 |
| Potassium dihydrogen phosphate | 1 |
| Sodium chloride | 2 |
| Magnesium sulphate | 0.1 |
| Agar | 20 |
| Sterilise at 116°C and Cool to 50°C | |
| 50 % egg yolk in sterile saline | 5 ml |

Ce milieu est séparé en 6 fractions. A chacune de ces fractions est ajouté l'un des substrats suivants : Magenta-β-glucoside (80mg/l), Alizarine-β-glucoside (50mg/l), CHE-β-glucoside (300mg/l), 8-Hydroxiquinoline-β-glucoside (300mg/l), DHF-β-glucoside (300mg/l), Blue-β-glucoside (150mg/l). Les substrats étaient des substrats commerciaux obtenus auprès d'entreprises spécialisées dans la fourniture de substrats enzymatiques synthétiques. Le substrat à base d'alizarine était synthétisé selon le protocole décrit dans la demande de brevet EP1235928. Le substrat à base de CHE était synthétisé selon les protocoles décrits dans la demande de brevet EP0900230.

### 2. ESSAIS

Les milieux sont répartis en boites de Petri.
L'ensemencement est effectué à partir de pré-cultures de 48h à 37°C en anaérobiose. Une suspension en eau physiologique à 0,5 McF est réalisée puis 1µl de chaque suspension est déposé sur chaque boite.
Des lectures sont effectuées après 48h d'incubation.

### 3. RESULTATS

| | | Magenta-β-Glucoside (80 mg/L) | | Alizarine-β-Glucoside (50 mglL) | | CHE-β-Glucoside (300 mg/L)¹ | | DHF-β-Glucoside (300 mg/L)² | |
|---|---|---|---|---|---|---|---|---|---|
| souches | Temps d'incubation | Cr | Co | Cr | Co | Cr | Co | Cr | Co |
| *Clostridium difficile* | 48h | 18/28 | 16/18 | 22/28 | 18/22 | 21/28 | 20/21 | 28/28 | 20/28 |
| Autres *Clostridium* | 48h | 17/17 | 6/17 | 16/17 | 2/16 | 17/17 | 6/17 | 12/14 | 3/12 |

| | | | | | | | | | |
|---|---|---|---|---|---|---|---|---|---|
| Cr = croissance ; Co = coloration ¹ = + 500mg/L de citrate de fer ammoniacal ² = + 50 mg de citrate de fer ammoniacal | | | | | | | | | |

### 4. INTERPRETATION

Tous les substrats permettaient la mise en évidence de *C difficile.* L'Alizarine-β-glucoside, le Magenta-β-glucoside (5-Bromo-6-chloro-3-indoxyl-β-glucoside) et, en particulier, le CHE-β-Glucoside (3,4-Cyclohexenoesculetine-β-glucoside) présentaient une bonne sensibilité vis à vis des *Clostridium difficile.*

### Exemple 3

Des souches du genre *Clostridium spp* ont été testées sur 4 milieux différents contenant chacun un substrat chromogénique de beta-glucosidase et 300 mg/L de citrate de fer ammoniacal, selon un protocole similaire a celui décrit dans l'exemple 2. La base des milieux était une base Colombia, et les substrats testés étaient les suivants :
- Blue-β-glucoside - (5-Bromo-3- indoxyl-β-glucoside) à une concentration de 300 mg/l
- 8HQ-βglucoside -(8-Hydroxyquinoline-β-glucoside) à une concentration de 150 mg/l)
- Alizarineβ-glucoside (1,2- Dihydroxyanthraquinone-β-glucoside) à une concentration de 50 mg/l
- DHF-β-glucoside à une concentration de 300 mg/l
La lecture des boites est ensuite effectuée à 48h afin de déterminer la spécificité de chaque substrat.
Les résultats sont présentés ci dessous :

| | | DHF-β-Glucoside (300 mg/L) | | Alizarine-β-Glucoside (50 mg/L) | | 8-Hydroxyquinoline-β-Glucoside (150 mg/L) | | Blue-β-Glucoside (300 mg/L) | |
|---|---|---|---|---|---|---|---|---|---|
| souches | Temps d'incubation | Cr | Co | Cr | Co | Cr | Co | Cr | Co |
| *Clostridium difficile* | 48h | 10/10 | 10/10 | 10/10 | 8/10 | 10/10 | 5/10 | 10/10 | 10/10* |

| | | | | | | | | | |
|---|---|---|---|---|---|---|---|---|---|
| Cr = croissance ; Co = coloration ; * = diffusion de la coloration | | | | | | | | | |

Tous les substrats permettaient la mise en évidence de *C difficile.*

### Exemple 4

### 1. MILIEU ET MICROORGANISMES

Cinq milieux contenant chacun 48,10 g/l de base Columbia, 2,5 g/l de Taurocholate de sodium, 200 mg/l de Citrate de fer ammoniacal, 100 mg/l de CHE-beta-Glucoside, ainsi qu'un système inhibiteur habituellement utilisé dans les milieux pour *Clostridium difficile* à base de D-Cyclosérine et des concentrations variables en Esculine et Glucose (voir tableau suivant)

| | milieu 1 | milieu 2 | milieu 3 | milieu 4 | milieu 5 |
|---|---|---|---|---|---|
| Glucose g/l | 1 | 0 | 1 | 1 | 1 |
| Esculine mg/l | 0 | 37,5 | 25 | 37,5 | 50 |

### 1. ESSAI

L'ensemencement est effectué à partir de pré-cultures de 48h à 37°C en anaérobiose. Une suspension en eau physiologique à 0,5 McF est réalisée puis les souches sont ensemencées à l'oese calibrée de 10µl.
Les lectures sont effectuées après 24 heures d'incubation à 37°C.

### 2. RESULTATS

Pour la croissance :
• Les valeurs indiquées correspondent au diamètre des colonies
Pour l'intensité de coloration :
- 1 correspond à la présence d'une coloration nette de faible intensité
- 1,5 correspond à la présence d'une coloration intermédiaire aux colorations 1 et 2,
- 2 correspond à la présence d'une coloration franche d'intensité moyenne,
- 2,5 correspond à la présence d'une coloration intermédiaire aux colorations 2 et 3,
- 3 correspond à la présence d'une coloration intense.

**C = Croissance, I = Intensité de coloration, * = diffusion de la coloration**

| | | Milieu 1 | | Milieu 2 | | Milieu 3 | | Milieu 4 | | Milieu 5 | |
|---|---|---|---|---|---|---|---|---|---|---|---|
| | | C | 1 | C | I | C | I | C | I | C | I |
| *C. difficile 042* | 24h | 2,5 | 2 | 1 | 2,5 | 2 | 2,5 | 2 | 2,5* | 2 | 2,5* |
| *C. difficile 308* | 24h | 1,5 | 2 | 1 | 3 | 1,5 | 3 | 1,5 | 3* | 1,5 | 3* |
| *C. difficile 169* | 24h | 1,5 | 1 | 1,5 | 2 | 1,5 | 1,5 | 1,5 | 1,5* | 1,5 | 1,5* |
| *C. difficile 187* | 24h | 1,25 | 2 | 0,75 | 2 | 1,25 | 2,5 | 1,25 | 2,5* | 1,5 | 2,5* |

### 4. INTERPRETATION

D'après le milieu 2, l'absence de glucose entraîne une diminution de la taille des colonies et l'esculine quant à elle, a un effet positif sur l'intensité de la coloration.
Dans les milieux 3, 4, et 5, les 2 composés ont été mélangés. On note pour les milieux 4 et 5 une diffusion plus ou moins importante de la coloration dans la gélose.

Le milieu 3 représente donc le meilleur compromis entre l'amélioration de la croissance et de l'intensité de coloration.

### Exemple 5

### 1. MILIEU ET MICROORGANISMES

107 souches ont été testées sur deux milieux prototypes pour C. *difficile* dont les compositions sont les suivantes (pour un litre):

| | Milieu 1 | Milieu 2 |
|---|---|---|
| Base Columbia | 48,10 g | 48,10 g |
| Glucose | 1,25 g | 1 g |
| Taurocholate | 2,5 g | 2,5 g |
| Citrate de fer ammoniacal | 0,2 g | 0,2 g |
| Autoclavage de la base et ajout des additifs | | |
| CHE-β-Glucoside | 0,100 g | 0,100 g |
| Esculine | / | 0,025 g |

| | | |
|---|---|---|
| Système sélectif dans les 2 milieux selon George et al « Selective and differential medium for isolation of Clostridium difficile », Journal of Clinical Microbiology vol 9, p214-219, 1979. | | |

### 2. ESSAIS

L'ensemencement est effectué à partir de pré-cultures de 48h à 37°C en anaérobiose.
Une suspension en eau physiologique à 0,5 McF est réalisée puis les souches sont ensemencées à l'oese calibrée de 10µl.
Les lectures sont effectuées après 24 et 48 heures d'incubation à 37°C.

### 3. RESULTATS

| | Total souches | Temps d'incubation | % de souches produisant une coloration | |
|---|---|---|---|---|
| | | | **Milieu 1** | **Milieu 2** |
| *Clostridium difficile* | 31 | 24h | **90** | **90** |
| | | 48h | **94** | **94** |
| Autres *Clostridium* | 34 | 24h | 3 | 3 |
| | | 48h | 3 | 6 |
| Autres souches (Gram⁺, Gram⁻) | 42 | 24h | 5 | 9 |
| | | 48h | 12 | 32 |
| | 107 | | | |

### 4. INTERPRETATION

Le substrat CHE-β-glucoside présente une bonne sensibilité vis à vis des *Clostridium difficile* dès 24h. La sélectivité du milieu est elle aussi très satisfaisante puisqu'à 24h seules 8 souches sur 76, autres que *Clostridium difficile*, poussent sur le milieu 1. Il permet d'augmenter l'intensité de coloration des *Clostridium difficile* et d'homogénéiser des souches qui ont un aspect hétérogène sur le milieu 1.
En conclusion, le substrat CHE-β-glucoside présente une bonne spécificité et une bonne sensibilité vis à vis des *Clostridium difficile* puisqu'il permet de mettre en évidence près de 90% des souches avec un minimum de faux-positifs dès 24h.

### Exemple 6.

Des prélèvements de selles de patients susceptibles d'être infectés par une souche de *Clostridium difficile* ont été testés sur 4 milieux différents.

### 1. MILIEU ET MICROORGANISMES

Le premier milieu (A) correspond au milieu de l'exemple 1 auquel a été ajouté de la D-Cyclosérine (250mg/l) et de la Céfoxitine (16mg/l). Le deuxième milieu (B) correspond au milieu 1 de l'exemple 5. Les 3^{e} (C) et 4^{e} (D) milieux sont basés sur le milieu chromID Salmonella de la demanderesse, dépourvu de ses substrats enzymatiques, additionné de Cystéine à 0,5g/l et dont le système sélectif a été remplacé par celui du milieu 1 de l'exemple 5. Le milieu C comprend en outre du CHE-glucoside à 100mg/l du citrate de fer ammoniacal à 200mg/l, du Taurocholate de sodium à 2,5g/l et du Glucose à 1,25g/l. Le milieu D comprend en outre du CHE-glucoside à 300mg/l du citrate de fer ammoniacal à 500mg/l, du Taurocholate de sodium à 1g/l et du Sérum de cheval à 3%.

### 2. ESSAIS

Les milieux sont répartis en boites de Petri.
L'ensemencement est effectué à partir de prélèvements de selles homogénéisés. Des lectures sont effectuées après 48h d'incubation.

### 3. RESULTATS

| | Milieu A | | Milieu B | | Milieu C | | Milieu D | |
|---|---|---|---|---|---|---|---|---|
| | Nb¹ | Couleur | Nb | Couleur | Nb | Couleur | Nb | Couleur |
| *Clostridium difficile* | 13 | Noire | 12 | Noire ou à centre gris | 14 | Grise | 14 | Noire |
| Autres | 2 | Noire | 2 | Noire | 10 | Incolore à blanc | 8 | Incolore à blanc |
| | 15 | Incolore à blanc | 7 | Incolore à blanc | | | | |

| | | | | | | | | |
|---|---|---|---|---|---|---|---|---|
| 1 : Nb = Nombre de microorganismes détectés | | | | | | | | |

### 4. INTERPRETATION

Ces quatre milieux ont permis une détection aisée des souches de *Clostridium difficile* présentes dans des prélèvements plurimicrobiens comme les selles. De façon surprenante, la détection a été encore plus aisée sur les milieux C et D qui sont pourtant basés sur un milieu pour bactéries à métabolisme aérobie facultatif. Cependant la taille moyenne des colonies de *C. difficile* était supérieure sur le milieu A (3,8mm) que sur les milieux B, C et D (respectivement 1,6 ; 2,1 et 2 mm). Pour les milieux C et D, cette moindre croissance était compensée par une meilleure sélectivité (inhibition totale ou partielle des autres microorganismes) et une meilleure spécificité, aucune souche n'appartenant pas à l'espèce *C. difficile* ne produisant des colonies noires.
La variation de la concentration du substrat de beta-glucosidase entre les milieux C et D entraîne une modification du contraste de coloration sans perturber les performances du milieu en terme de nombre de souches correctement détectées.

## Revendications

1. Procédé de détection et/ou d'identification de *Clostridium difficile*, **caractérisé en ce qu'**il comprend les étapes suivantes :
a) disposer d'un milieu réactionnel comprenant au moins un substrat de beta-glucosidase, apte à l'identification de *C. difficile*,
b) ensemencer le milieu avec un échantillon biologique à tester,
c) laisser incuber, et
d) détecter l'hydrolyse du substrat de beta-glucosidase indiquant la présence de *Clostridium difficile.*

2. Procédé de détection et/ou d'identification de *Clostridium difficile*, selon la revendication 1, selon lequel, lors de l'étape c), l'incubation est effectuée en anaérobiose.

3. Procédé de détection et/ou d'identification de *Clostridium difficile*, selon la revendication 1 ou 2 selon lequel ledit substrat de beta-glucosidase est choisi parmi l'Alizarine-β-glucoside, le Magenta-β-glucoside (5-Bromo-6-chloro-3-indoxyl-β-glucoside) et le CHE-β-Glucoside (3,4-Cyclohexenoesculetine-β-glucoside).

4. Procédé de détection et/ou d'identification de *Clostridium difficile,* selon l'une quelconque des revendications 1 à 3 selon lequel ledit substrat de beta-glucosidase est à une concentration comprise entre 25 et 1000mg/l, plus préférentiellement entre 50 et 400mg/l.

5. Procédé de détection et/ou d'identification de *Clostridium difficile,* selon l'une quelconque des revendications 1 à 4 selon lequel ledit milieu réactionnel comprend en outre, de l'esculine.

6. Procédé de détection et/ou d'identification de *Clostridium difficile*, selon la revendication 5 selon lequel l'esculine est à une concentration comprise entre 5 et 500 mg/l, préférentiellement entre 10 et 100 mg/l.

7. Procédé de détection et/ou d'identification de *Clostridium difficile*, selon l'une quelconque des revendications 1 à 6 selon lequel ledit milieu réactionnel comprend en outre, un inducteur de β-glucosidase.

8. Procédé de détection et/ou d'identification de *Clostridium difficile*, selon l'une quelconque des revendications 1 à 7 selon lequel ledit milieu réactionnel comprend en outre, au moins un activateur de croissance des souches de *C. difficile*.

9. Procédé de détection et/ou d'identification de *Clostridium difficile*, selon l'une quelconque des revendications 1 à 8 selon lequel ledit milieu réactionnel comprend en outre, au moins un inducteur de la germination des spores de *C. difficile.*

10. Procédé de détection et/ou d'identification de *Clostridium difficile*, selon l'une quelconque des revendications 1 à 9 selon lequel ledit milieu réactionnel comprend en outre au moins un agent sélectif.

11. Procédé de détection et/ou d'identification de *Clostridium difficile*, selon l'une quelconque des revendications 1 à 10 selon lequel ledit milieu réactionnel comprend en outre au moins un deuxième substrat enzymatique.

12. Procédé de détection et/ou d'identification de *Clostridium difficile*, selon l'une quelconque des revendications 1 à 11 selon lequel ledit milieu réactionnel comprend en outre au moins un agent réducteur.

13. Utilisation d'un milieu réactionnel comprenant au moins un substrat de beta-glucosidase, pour la détection et/ou l'identification de *Clostridium difficile.*

14. Utilisation d'un milieu réactionnel selon la revendication 13 **caractérisé en ce que** ledit substrat de beta-glucosidase est choisi parmi l'Alizarine-β-glucoside, le Magenta-β-glucoside (5-Bromo-6-chloro-3-indoxyl-β-glucoside) et le CHE-β-Glucoside (3,4-Cyclohexenoesculetine-β-glucoside).

15. Utilisation d'un milieu réactionnel selon la revendication 13 ou 14 **caractérisé en ce que** ledit substrat de beta-glucosidase est à une concentration comprise entre 25 et 1000mg/l et plus préférentiellement entre 50 et 400mg/l.

16. Utilisation d'un milieu réactionnel selon l'une quelconque des revendications 13 à 15 **caractérisé en ce qu'**il comprend, en outre, de l'esculine.

17. Utilisation d'un milieu réactionnel selon la revendication 16 **caractérisé en ce que** l'esculine est à une concentration comprise entre 5 et 500mg/l.

18. Utilisation d'un milieu réactionnel selon l'une quelconque des revendications 13 à 17 **caractérisé en ce qu'**il comprend, en outre, au moins un inducteur de ß-glucosidase.

19. Utilisation d'un milieu réactionnel selon l'une quelconque des revendications 13 à 18 **caractérisé en ce qu'**il comprend, en outre, un ou plusieurs activateurs de croissance des souches de *C. difficile*.

20. Utilisation d'un milieu réactionnel selon l'une quelconque des revendications 13 à 19 **caractérisé en ce qu'**il comprend, en outre, au moins un inducteur de la germination des spores de *C. difficile*.

21. Utilisation d'un milieu réactionnel selon l'une quelconque des revendications 13 à 20 **caractérisé en ce qu'**il comprend, en outre, au moins un agent sélectif.

22. Utilisation d'un milieu réactionnel selon l'une quelconque des revendications 13 à 21 **caractérisé en ce qu'**il comprend, en outre, au moins un deuxième substrat enzymatique.

23. Utilisation d'un milieu réactionnel selon l'une quelconque des revendications 13 à 22 **caractérisé en ce qu'**il comprend, en outre, au moins un agent réducteur.

## Patentansprüche

1. Ein Verfahren zum Nachweis und/oder zur Identifikation von *Clostridium difficile*, **dadurch gekennzeichnet, dass** es die folgenden Schritte beinhaltet:
a) Verfügen über ein Reaktionsmedium, das mindestens ein Beta-Glucosidase-Substrat beinhaltet, das sich für die Identifizierung von *C. difficile* eignet,
b) Impfen des Mediums mit einer zu testenden biologischen Probe,
c) Inkubierenlassen und
d) Nachweisen der Hydrolyse des Beta-Glucosidase-Substrats, die das Vorhandensein von *Clostridium difficile* anzeigt.

2. Verfahren zum Nachweis und/oder zur Identifikation von *Clostridium difficile* gemäß Anspruch 1, wobei die Inkubation während des Schritts c) unter anaeroben Bedingungen erfolgt.

3. Verfahren zum Nachweis und/oder zur Identifikation von *Clostridium difficile* gemäß Anspruch 1 oder 2, wobei das Beta-Glucosidase-Substrat aus Alizarin-β-glucosid, Magenta-β-glucosid (5-Brom-6-chlor-3-indoxyl-β-glucosid) und CHE-β-glucosid (3,4-Cyclohexenesculetin-β-glucosid) ausgewählt wird.

4. Verfahren zum Nachweis und/oder zur Identifikation von *Clostridium difficile* gemäß einem der Ansprüche 1 bis 3, wobei das Beta-Glucosidase-Substrat in einer Konzentration zwischen 25 und 1000 mg/l, mehr bevorzugt zwischen 50 und 400 mg/l vorliegt.

5. Verfahren zum Nachweis und/oder zur Identifikation von *Clostridium difficile* gemäß einem der Ansprüche 1 bis 4, wobei das Reaktionsmedium zudem Esculetin beinhaltet.

6. Verfahren zum Nachweis und/oder zur Identifikation von *Clostridium difficile* gemäß Anspruch 5, wobei das Esculetin in einer Konzentration zwischen 5 und 500 mg/l, vorzugsweise zwischen 10 und 100 mg/l vorliegt.

7. Verfahren zum Nachweis und/oder zur Identifikation von *Clostridium difficile* gemäß einem der Ansprüche 1 bis 6, wobei das Reaktionsmedium zudem einen β-Glucosidase-Induktor beinhaltet.

8. Verfahren zum Nachweis und/oder zur Identifikation von *Clostridium difficile* gemäß einem der Ansprüche 1 bis 7, wobei das Reaktionsmedium zudem mindestens einen Wachstumsaktivator für die Stämme von C. *difficile* beinhaltet.

9. Verfahren zum Nachweis und/oder zur Identifikation von *Clostridium difficile* gemäß einem der Ansprüche 1 bis 8, wobei das Reaktionsmedium zudem mindestens einen Induktor für die Keimbildung der Sporen von *C. difficile* beinhaltet.

10. Verfahren zum Nachweis und/oder zur Identifikation von *Clostridium difficile* gemäß einem der Ansprüche 1 bis 9, wobei das Reaktionsmedium zudem mindestens ein selektives Mittel beinhaltet.

11. Verfahren zum Nachweis und/oder zur Identifikation von *Clostridium difficile* gemäß einem der Ansprüche 1 bis 10, wobei das Reaktionsmedium zudem mindestens ein zweites Enzymsubstrat beinhaltet.

12. Verfahren zum Nachweis und/oder zur Identifikation von *Clostridium difficile* gemäß einem der Ansprüche 1 bis 11, wobei das Reaktionsmedium zudem mindestens ein Reduktionsmittel beinhaltet.

13. Eine Verwendung eines Reaktionsmediums, das mindestens ein Beta-Glucosidase-Substrat für den Nachweis und/oder die Identifikation von *Clostridium difficile* beinhaltet.

14. Verwendung eines Reaktionsmediums gemäß Anspruch 13, **dadurch gekennzeichnet, dass** das Beta-Glucosidase-Substrat aus Alizarin-β-glucosid, Magenta-β-glucosid (5-Brom-6-chlor-3-indoxyl-β-glucosid) und CHE-β-glucosid (3,4-Cyclohexenesculetin-β-glucosid) ausgewählt wird.

15. Verwendung eines Reaktionsmediums gemäß Anspruch 13 oder 14, **dadurch gekennzeichnet, dass** das Beta-Glucosidase-Substrat in einer Konzentration zwischen 25 und 1000 mg/l und mehr bevorzugt zwischen 50 und 400 mg/l vorliegt.

16. Verwendung eines Reaktionsmediums gemäß einem der Ansprüche 13 bis 15, **dadurch gekennzeichnet, dass** es zudem Esculetin beinhaltet.

17. Verwendung eines Reaktionsmediums gemäß Anspruch 16, **dadurch gekennzeichnet, dass** das Esculetin in einer Konzentration zwischen 5 und 500 mg/l vorliegt.

18. Verwendung eines Reaktionsmediums gemäß einem der Ansprüche 13 bis 17, **dadurch gekennzeichnet, dass** es zudem mindestens einen β-Glucosidase-Induktor beinhaltet.

19. Verwendung eines Reaktionsmediums gemäß einem der Ansprüche 13 bis 18, **dadurch gekennzeichnet, dass** es zudem einen oder mehrere Wachstumsaktivatoren für die Stämme von *C. difficile* beinhaltet.

20. Verwendung eines Reaktionsmediums gemäß einem der Ansprüche 13 bis 19, **dadurch gekennzeichnet, dass** es zudem mindestens einen Induktor für die Keimbildung der Sporen von *C. difficile* beinhaltet.

21. Verwendung eines Reaktionsmediums gemäß einem der Ansprüche 13 bis 20, **dadurch gekennzeichnet, dass** es zudem mindestens ein selektives Mittel beinhaltet.

22. Verwendung eines Reaktionsmediums gemäß einem der Ansprüche 13 bis 21, **dadurch gekennzeichnet, dass** es zudem mindestens ein zweites Enzymsubstrat beinhaltet.

23. Verwendung eines Reaktionsmediums gemäß einem der Ansprüche 13 bis 22, **dadurch gekennzeichnet, dass** es zudem mindestens ein Reduktionsmittel beinhaltet.

## Claims

1. A method for detecting and/or identifying *Clostridium difficile*, **characterized in that** it comprises the following steps:
a) providing a reaction medium comprising at least one beta-glucosidase substrate capable of identifying *C. difficile*,
b) inoculating the medium with a biological sample to be tested,
c) allowing for incubation, and
d) detecting the hydrolysis of the beta-glucosidase substrate, indicative of the presence of *Clostridium difficile*.

2. The method for detecting and/or identifying *Clostridium difficile*, as claimed in claim 1, wherein, during step c), the incubation is carried out under anaerobic conditions.

3. The method for detecting and/or identifying *Clostridium difficile*, as claimed in claim 1 or 2, wherein said beta-glucosidase substrate is chosen from alizarin-β-glucoside, magenta-β-glucoside (5-bromo-6-chloro-3-indoxyl-β-glucoside) and CHE-β-glucoside (3,4-cyclohexenoesculetin-β-glucoside).

4. The method for detecting and/or identifying *Clostridium difficile*, as claimed in any one of claims 1 to 3, wherein said beta-glucosidase substrate is at a concentration of between 25 and 1000 mg/l, more preferably between 50 and 400 mg/l.

5. The method for detecting and/or identifying *Clostridium difficile*, as claimed in any one of claims 1 to 4, wherein said reaction medium further comprises esculin.

6. The method for detecting and/or identifying *Clostridium difficile,* as claimed in claim 5, wherein the esculin is at a concentration of between 5 and 500 mg/l, preferably between 10 and 100 mg/l.

7. The method for detecting and/or identifying *Clostridium difficile*, as claimed in any one of claims 1 to 6, wherein said reaction medium further comprises a ß-glucosidase inducer.

8. The method for detecting and/or identifying *Clostridium difficile*, as claimed in any one of claims 1 to 7, wherein said reaction medium further comprises at least one growth activator for *C. difficile* strains.

9. The method for detecting and/or identifying *Clostridium difficile*, as claimed in any one of claims 1 to 8, wherein said reaction medium further comprises at least one *C. difficile* spore germination inducer.

10. The method for detecting and/or identifying *Clostridium difficile*, as claimed in any one of claims 1 to 9, wherein said reaction medium further comprises at least one selective agent.

11. The method for detecting and/or identifying *Clostridium difficile*, as claimed in any one of claims 1 to 10, wherein said reaction medium further comprises at least a second enzyme substrate.

12. The method for detecting and/or identifying *Clostridium difficile*, as claimed in any one of claims 1 to 11, wherein said reaction medium further comprises at least one reducing agent.

13. Use of a reaction medium comprising at least a beta-glucosidase substrate, for detecting and/or identifying *Clostridium difficile*.

14. The use of a reaction medium according to claim 13, wherein said beta-glucosidase substrate is selected from the group consisting of alizarin-beta-glucoside, magenta-beta-glucoside (5-bromo-6-chloro-3-indoxyl-beta-glucoside) and CHE-beta-glucoside (3,4-cyclohexenoesculetin-beta-glucoside).

15. The use of a reaction medium according to claim 13 or 14 wherein said beta-glucosidase substrate is at a concentration of between 25 and 1000 mg/l, and more preferably between 50 and 400 mg/l.

16. The use of a reaction medium according to any one of the claims 13 to 15 wherein said reaction medium further comprises esculin.

17. The use of a reaction medium according to claim 16 wherein the esculin is at a concentration of between 5 and 500 mg/l.

18. The use of a reaction medium according to any one of claims 13 to 17, wherein said reaction medium further comprises a beta-glucosidase inducer.

19. The use of a reaction medium according to any one of claims 13 to 18 wherein said reaction medium further comprises at least one growth activator for *C. difficile* strains.

20. The use of a reaction medium according to any one of claims 13 to 19, wherein said reaction medium further comprises at least one *C. difficile* spore germination inducer.

21. The use of a reaction medium according to any one of claims 13 to 20, wherein said reaction medium further comprises at least one selective agent.

22. The use of a reaction medium according to any one of claims 13 to 21, wherein said reaction medium further comprises at least a second enzyme substrate.

23. The use of a reaction medium according to any one of claims 13 to 22, wherein said reaction medium further comprises at least one reducing agent.
